# EUROPEAN PATENT APPLICATION

(11) **EP 4 390 962 A1**
(43) Date of publication of application: **26.06.2024**
(21) Application number: 22214811.6
(22) Date of filing: 20.12.2022
(51) Int. Cl.: G16H 40/40

(54) **MEDICAL DEVICE FAULT PREDICTION**

(71) Applicant: Koninklijke Philips N.V., 5656 AG Eindhoven (NL)
(72) Inventor: SOUBHIK, Paul, Eindhoven (NL); PATIL, Meru Adagouda, Eindhoven (NL); JOHNSON, Mark Thomas, 5656AG Eindhoven (NL)
(74) Representative: Philips Intellectual Property & Standards

(57) **Abstract**

Proposed are concepts pertaining to aiding fault resolution through the identification of new sets of factors that may contribute to the occurrence of one or more faults. In particular, embodiments of the invention propose predicting or deriving un-foreseen faults from existing or known faults based on the associated parameters, events, etc. (i.e. contributing factors). By identifying un-foreseen/un-documented faults, solutions may be identified prior to occurrence of problems, potentially reducing resolution/fixing time, cost and downtime of medical equipment.

## Description

### FIELD OF THE INVENTION

The present invention relates to the field of equipment maintenance, and in particular to the field of predicting faults of a medical device.

### BACKGROUND OF THE INVENTION

A medical imaging device may occasionally malfunction, i.e., exhibit a fault and fail to work properly.

Typically, in response to a fault in a medical imaging device, one or multiple service engineers are assigned to diagnose and resolve the fault. Such service engineers may perform the diagnosis remotely, or on-site.

In such a workflow, it is not always possible to list all the problems/issues that may occur in a medical system during development and prepare a corresponding solution. When such un-foreseen problems arise, significant time and effort from the service engineers needs to be devoted to diagnosing and solving the issue.

There is therefore a desire to reduce the length of time taken to identify the cause of, or solution to, a fault in a medical imaging device.

### SUMMARY OF THE INVENTION

The invention is defined by the claims.
According to examples in accordance with an aspect of the invention, there is provided a method for predicting faults of a medical device, the method comprising:
obtaining a first set of contributing factors that led to a first fault of the medical device;
inputting a description of the first fault of the medical device and the first set of contributing factors to a machine learning algorithm, the machine learning algorithm being trained to predict a predicted second set of contributing factors that will lead to an occurrence of the first fault of the medical device, the second set of contributing factors being different to the first set of contributing factors; and
outputting the second set of contributing factors.

Proposed concepts thus aim to provide schemes, solutions, concepts, designs, methods and systems pertaining to aiding fault resolution through the identification of new sets of factors that may contribute to the occurrence of one or more faults. In this way, un-foreseen/un-documented problems/faults that may occur during the usage of the medical equipment/devices can be identified a-priori.

Embodiments may predict or derive un-foreseen faults from existing or known faults based on the associated (log file) parameters, events, etc. (i.e. contributing factors). Examples of associated log file parameters include the values of settings used to control the medical device, and the values of monitored currents, voltages, temperatures, speeds, and so forth that are monitored during the operation of the medical device. The values of the log file parameters may be extracted from a log file of the medical device. By identifying un-foreseen/un-documented problems/faults, solutions may be identified prior to occurrence of problems, potentially reducing resolution/fixing time, cost and downtime of medical equipment.

The proposed concept(s) may also enhance reliability of the medical device development phase by providing information about faults and associated solutions that can be leverage to help avoid or prevent occurrence of the faults in the device usage phase.

In particular, embodiments of the invention propose using a first set of contributing factors that led to a first fault of a medical device to generate (e.g. predict) a second, different set of contributing factors that will lead to an occurrence of the first fault. The second, different set of contributing factors may be used to identify one or more new faults that may be related or linked to the first fault. That is, embodiments may generate information that can be used to identify linked or related faults, and solutions to these faults may then be determined prior to their occurrence in the field, for example.

A proposed concept recognizes that slight changes the contributing factors of a fault may result in one more different but linked or similar faults. By predicting, from a first set of contributing factors of a fault, a second, different set that will lead to occurrence of the same faut, variants of the fault (or similar faults) may be identified in order to assist in fault diagnosis and resolution. It is also proposed that the relevance of contributing factors to a fault may be learnt (e.g. from historical data) and thus used to predict alternative sets of contributing factors using machine-learning.

Embodiments may therefore simplify the job of exploring and resolving potential faults for a medical device, by assisting the exploration of linked or related faults in a guided manner. Such embodiments may provide the advantages of reduced search/exploration time and improved (i.e. reduced) fault resolution time.

In other words, embodiments propose to use machine learning to generate sets of contributing factors for faults of the medical imaging device. Such sets of contributing factors may be used to identify new/unforeseen faults that are linked and/or similar to existing/known faults, thus facilitating early or pre-emptive identification of one or more solutions. Predictions provided by embodiment may thus aid fault analysis and/or diagnosis prior to field deployment of a medical device. Accordingly, embodiments may be used in relation to fault resolution so as to support a service and/or development engineers when diagnosing and resolving faults. Improved (e.g. quicker) fault resolution in medical imaging devices may therefore be provided by proposed concepts.

Embodiments may further comprise: analyzing the second set of contributing factors and the first fault of the medical device to determine if the second set of contributing factors leads to an occurrence of the first fault; and responsive to determining that the second set of contributing factors does not lead to an occurrence of the first fault, determining a predicted fault of the medical device that is led to by the second set of contributing factors, wherein the predicted fault is different from the first fault.

By way of example, the step of analyzing may comprise: determining a value of a similarity measure between the first set of contributing factors attributes and the second set of contributing factors; and based on the determining value of a similarity measure, determining if the second set of contributing factors leads to an occurrence of the first fault. The similarity measure may be the Cosine similarity metric, the Euclidian distance, or the Jaccard similarity coefficient, for example.

In another example, the step of analyzing may comprise: representing the first and second set of contributing factors and the first fault as connected graphs; comparing the connected graphs; and based on the comparison result, determining if the second set of contributing factors leads to an occurrence of the first fault.

Some embodiments may further comprise: determining a solution to the predicted fault of the medical device.

The machine learning algorithm may be trained using a training algorithm configured to receive an array of training inputs and respective known outputs, wherein a training input comprises: problem data describing a known fault of the medical device and an associated set of contributing factors that lead to an occurrence of the known fault of the medical device; and wherein a respective known output comprises a different set contributing factors that lead to an occurrence of the known fault of the medical device. In this way, the machine learning algorithm may be trained to output a new, different set of contributing factors for a fault of a medical device when provided with a description of the fault and a set of contributing factors that led to the fault. Previous information regarding faults may thus be learnt and leveraged to make future suggestions for contributing factors of same (or similar) problems/faults.

In some embodiments, obtaining the first set of contributing factors may comprise: obtaining problem data describing the fault of the medical device; and analyzing the problem data to determine a first set of contributing factors that led to the fault of the medical device. In this way, actions and steps of a user may be automatically monitored and recorded so as to provide data for use with the machine learning algorithm. A need for a service engineer to actively record and/or provide information about previous interaction(s) may therefore be avoided/alleviated by proposed embodiments.

The process of obtaining the first set of contributing factors may comprise analyzing an event history of the medical device. That is, embodiments may take advantage of logged/monitored actions to obtain information about factors that led to occurrence of a fault. Existing/conventional log file applications may therefore be leveraged to facilitate the implementation of proposed embodiments.

In some embodiments, obtaining the first set of contributing factors may comprise: receiving, via in input interface, a description of contributing factors provided by a respondent in response to a request for data. The request for data may, for example, comprise a fault analysis questionnaire, or employ old maintenance records commented in earlier similar failure. In this way, responses of the respondent (e.g. end user or service engineer) to the questionnaire may be provided as data for identifying a first set of contributing factors. Such responses may thus be used to identify one or more contributing factors of the first fault. Embodiments may therefore cater for natural language description of a medical imaging device fault, thereby facilitating simple and intuitive data provision.

In an embodiment, the step of obtaining the first set of contributing factors may comprise performing a natural language processing analysis on the description of the fault of the medical device. By catering for natural language description of a medical imaging device fault and/or its contributing factors, embodiments may facilitate the use of data that has been provided/recorded in a simple and intuitive manner.

In some embodiments, the machine learning algorithm may comprise a generative adversarial network.

One or more concepts for predicting/deriving un-foreseen faults/problems from known faults are thus provided, and these may enable the pre-emptive identification of one or more solutions to save time, cost and downtime of medical equipment.

According to another aspect, there is provided a computer program product, wherein the computer program product comprises a computer-readable storage medium having computer-readable program code embodied therewith, the computer-readable program code configured to perform all of the steps of a proposed embodiment.

Thus, there may also be provided a computer system comprising: a computer program product according to proposed embodiment; and one or more processors adapted to perform a method according to a proposed concept by execution of the computer-readable program code of said computer program product.

According to another aspect of the invention, there is system for predicting faults of a medical device, the system comprising:
a data interface configured to obtain a first set of contributing factors that led to a first fault of the medical device; and
a machine learning algorithm configured to receive a description of the first fault of the medical device and the first set of contributing factors, the machine learning algorithm being trained to output predicted second set of contributing factors that will lead to an occurrence of the first fault of the medical device.

The system may be remotely located from a user device for analyzing and/or diagnosing a medical device fault. In this way, a user (such as a service or development engineer) may have an appropriately arranged system that can receive information about contributing factors that will lead to an occurrence of a fault of the medical device at a location remotely located from the system. Embodiments may therefore enable a user to identify and/or explore unforeseen faults using a local system (which may, for example, comprise a portable display device, such as a laptop, tablet computer, mobile phone, PDA, etc.). By way of example, embodiments may provide an application for a mobile computing device, and the application may be executed and/or controlled by a user of the mobile computing device.

The system may further include: a server device comprising the system for predicting faults of a medical device; and a client device comprising a user-interface. Dedicated data processing means may therefore be employed for the purpose of predicting a second set of contributing factors that will lead to an occurrence of the first fault of the medical device, thus reducing processing requirements or capabilities of other components or devices of the system.

The system may further include a client device, wherein the client device comprises the data interface, machine learning algorithm, processor, and a display unit. In other words, a user (such as a service or development engineer) may have an appropriately arranged client device (such as a laptop, tablet computer, mobile phone, PDA, etc.) which processes received data in order to identify a predicted second set of contributing factors that will lead to an occurrence of the first fault of the medical device and generate a display control signal. Purely by way of example, embodiments may therefore provide a fault analysis system that enables identification of unforeseen faults that may be linked, similar or related to a first fault, wherein real-time communication between a user (e.g. service engineer) and a medical device is provided and can have its functionality extended or modified according to proposed concepts, for example.

It will be understood that processing capabilities may therefore be distributed throughout the system in different ways according to predetermined constraints and/or availability of processing resources.

One or more concepts are provided for predicting/deriving un-foreseen faults of a medical device problems, and this may support improved medical device fault diagnostics or resolution. By way of example, proposed embodiments may address the issue of having to wait for a fault to occur (e.g. after deployment of the device) before a solution can be determined. By identifying unforeseen problems and pre-emptively determining one or more solutions to such problems, downtime of medical equipment may be reduced or avoided.

These and other aspects of the invention will be apparent from and elucidated with reference to the embodiment(s) described hereinafter.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flow diagram of a method for predicting faults of a medical device according to an embodiment;
Fig. 2 depicts an overview of a proposed embodiment;
Fig. 3 depicts how the parameters, its values, events sequence tuple is combined with the noise vector as conditional parameters for the model of Figure 2;
Fig. 4 depicts a simplified block diagram of a system for predicting faults of a medical device according to an embodiment; and
Fig. 5 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

The invention proposes concepts for aiding fault diagnosis and/or fault resolution of a medical device. In particular, embodiments may provide a method and/or system which helps to identify un-foreseen faults from known faults and this may facilitate the identification of one or more solutions prior to occurrence of the fault(s). Proposed embodiments may therefore reduce or avoid downtime of medical equipment.

Proposed concepts may be based on a realization that a first set of contributing factors that led to a first fault of the medical device may be leveraged to identify a second set of contributing factors that are predicted to lead to an occurrence of the first fault of the medical device. With the second set of contributing factors being different, an actual fault caused by the second set of factors may be slightly different from the first fault and/or related/linked to the first fault. In this way, new, previously unseen or unpredicted faults and their contributing factors may be identified, thus facilitating pre-emptive action and fault resolution to undertaken.

Accordingly, embodiments may be used in relation to medical device fault analysis and/or diagnosis, particularly during a development phase (i.e. prior to deployment).

Also proposed is a concept for resolving a fault of a medical imaging device. This may be used to guide an engineer towards resolving a fault/issue.

Proposed embodiments may thus facilitate improved (e.g. quicker and/or easier) fault analysis and/or resolution through the identification of faults prior to their occurrence. Embodiments of the present invention are therefore directed toward enabling the improved searching of potential faults and their solution(s). Embodiment may provide an efficient and predictive fault exploration mechanism that enables prediction of unforeseen faults. A reduction in a time to diagnose and fixe a fault/problem may therefore be provided by proposed embodiments.

By way of further example, solutions to un-foreseen/un-documented faults that can occur during the usage of medical equipment can be prepared α-priori. These derived faults can be considered during the development and/or deployment phase of the medical equipment. Further, it may help to avoiding reoccurrence of faults in a subsequent generation of the medical device(s).

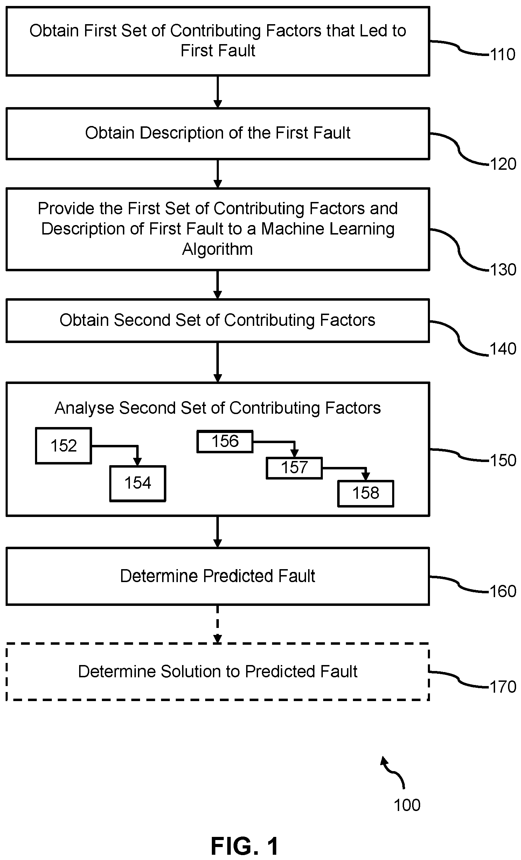

Fig. 1 illustrates an embodiment of a computer-implemented method 100 for predicting faults of a medical device. Specifically, the method leverages a first set of contributing factors that led to a first fault of a medical device to generate (e.g. predict) a second, different set of contributing factors that are precited to lead to occurrence of the first fault. The second, different set of contributing factors may be used to identify one or more new faults that may be related or linked to the first fault. That is, the embodiment generates information that can be used to identify linked or related faults, and solutions to these faults may then be determined prior to their occurrence.

The method begins with the step 110 of obtaining a first set of contributing factors that led to a first fault of the medical device. Here, the step 110 of obtaining the first set of contributing factors comprises analyzing an event history of the medical device. For instance, such embodiments may leverage functionality (e.g. an activity log/record) of a monitoring application to obtain information about previous events and actions.

However, in other embodiments, step 110 of obtaining the first set of contributing factors comprises: obtaining problem data describing the fault of the medical device; and analyzing the problem data to determine a first set of contributing factors that led to the fault of the medical device.

The method then proceeds to step 120 of obtaining problem data describing the first fault of the medical device. Specifically, the step 120 of obtaining problem input data comprises: receiving, via in input interface, problem data provided by a respondent in response to a request for data. In particular, the request for data comprises a fault analysis questionnaire provided to an engineer (e.g. via a user interface). Responses of the engineer to the questions of the questionnaire are thus obtained as initial problem input data and used to identify one or more features of the fault. Step 120 may also comprise performing a natural language processing (NLP) analysis on the problem data to identify one or more features of the first fault. The NLP analysis processes natural language description(s) of the fault, thereby facilitating the identification of features/aspects of the fault from information provided in a simple and intuitive manner. The description comprises a high-level definition of the reported fault. The description of the fault may include information such as an identification of a process executed by the medical device at the time of the fault, an outputted identification of the fault that is outputted by the medical device, or an error code outputted by the medical device at the time of the fault, for example.

At step 130, the first set of contributing factors and the problem data are provided to a neural network (NN)-based machine learning algorithm. The machine learning algorithm is trained to predict a second set of contributing factors that will lead to an occurrence of the first fault of the medical device, the second set of contributing factors being different to the first set of contributing factors.

The structure of an artificial NN (or, simply, neural network (NN)) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). However, the exemplary embodiment of Fig. 1 employs a generative adversarial network (GAN).

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

The training input data entries for the machine learning algorithm used in the method 100 correspond to example problem data and contributing factors. The training output data entries correspond to alternative contributing factors that lead to an occurrence of the fault of the medical device. That is, the machine learning algorithm is trained using a training algorithm configured to receive an array of training inputs and respective known outputs, wherein a training input comprises: problem data describing a known fault of the medical device and an associated set of contributing factors that lead to an occurrence of the known fault of the medical device; and wherein a respective known output comprises a different set contributing factors that lead to an occurrence of the known fault of the medical device. In this way, the machine learning algorithm is trained to output a second set of contributing factors that are predicted to lead to occurrence of a first fault when provided with a description of the first fault of a medical device and a first set of contributing factors that led to occurrence of the first fault.

At step 140, a prediction result is obtained from the machine learning algorithm. The prediction result comprises the predicted second set of contributing factors.

Next, step 150 comprises analyzing the second set of contributing factors and the first fault of the medical device to determine if the second set of contributing factors leads to an occurrence of the first fault. Here, step 150 may employ one or a range of alternative processes. By way of example, the flow diagram of Fig. 1 depicts two analysis processes that may be employed by the method.

The first exemplary analysis process comprises steps 152 and 154. Step 152 comprises determining a value of a cosine similarity measure between the first set of contributing factors attributes and the second set of contributing factors. Step 154 comprises determining if the second set of contributing factors leads to an occurrence of the first fault, based on the determining value of a cosine similarity measure. Step 154 may for instance involve determining a cosine similarity between the values of contributing factors such as the values of log file parameters.

The second exemplary analysis process comprises steps 156, 157 and 158. Step 156 comprises representing the first and second set of contributing factors and the first fault as connected graphs, and the connected graphs are compared in step 157. Step 158 then comprises determining if the second set of contributing factors leads to an occurrence of the first fault, based on the comparison result.

Finally, responsive to determining that the second set of contributing factors does not lead to an occurrence of the first fault, the method proceeds to step 160 of determining a predicted fault of the medical device that is led to by the second set of contributing factors, wherein the predicted fault is different from the first fault.

As illustrated by the dashed box in the flow diagram of Fig. 1, the method may further comprise the step 170 of determining a solution to the predicted fault of the medical device. The method may further comprise the step (not illustrated in Fig. 1) of resolving the predicted fault in accordance with the solution determined in the step 170.

Variations to the above-described embodiment of Fig. 1 may include employing different approaches to obtaining the first set of contributing factors. For example, in an alternative embodiment, obtaining the first set of contributing factors comprises: receiving, via in input interface, a description of contributing factors provided by a respondent in response to a request for data. The request for data may comprise a fault analysis questionnaire for example. In yet another embodiment, obtaining the first set of contributing factors comprises performing a natural language processing analysis on the description of the fault of the medical device.

According to the proposed concept(s), the main elements of an exemplary embodiment may be summarized as follows:
(i) For a known (first) problem of a medical device, identify the associated parameters and events (i.e. contributing factors) during the development phase of the medical device;
(ii) Use these parameters, combinations of different values of parameters, sequence of events (i.e. contributing factors) to identify new factors.
(iii) In step ii), a conditional Generative Network (DC-GAN) based technique is used to generate the new (second) factors. These may include a combination of the parameters that led to the first fault, or a similar unseen fault, yet with different values, and/or events/sequences of events that led to the same first fault, or a similar unseen fault;
(iv) Determine a solution to the newly discovered problem;
(v) Identify new paths that lead to a problem or discover new similar unseen problems, and use them during the deployment, with associated parameters in an actual problem logged;
(vi) Feed the new paths (i.e. contributing factors) of faults/problems as requirements to development phase in next iteration.

As discussed above, it is proposed to predict/derive unseen/new faults from a known fault set for medical equipment based on the associated parameters, sequence of events, etc. It is further proposed to prepare a solution to such an unseen/new fault, e.g. during a development phase so as to provide advance help to a service engineer

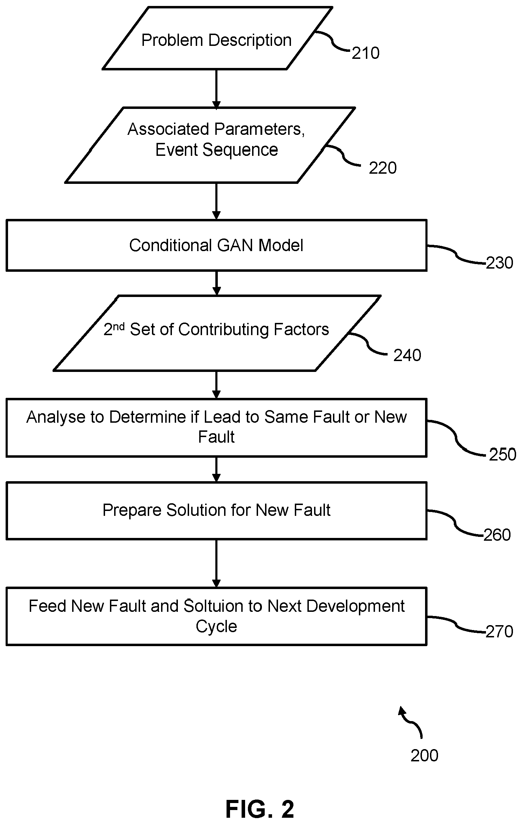

By way of a further example, Fig. 2 depicts an overview of a proposed embodiment. The steps of the proposed embodiment are as follows:
(i) Detection of parameters, events sequence, and conditions (i.e. contributing factors) for a known problem:
   First step is to understand a known/seen fault that is documented for the medical device. This may include identifying the underlying parameters, their values (in combination) and a sequence of the events that creates the ideal condition for the fault to occur. NLP techniques may be used to analyze such known, documented faults 210 and identify the factors 220 (e.g. parameters, range of values, events sequence etc.) that lead to the fault.

The problem description 210 is pre-processed and analyzed through a set of NLP techniques (such as tokenization, stemming, POS tagging, NER) and finally a named-entity set is generated to classify the words and phrases from the problem description text. Next, a system knowledge based is consulted for identifying the parameters 220 with their value ranges, events, and conditions (that leads to the fault).

(ii) Conditional Generative Networks to generate unseen problems:
Once the contributing factors (e.g. parameters, range of values, events sequence etc.) are extracted, next step is to generate new and unseen problem. This can be achieved through generative models where the known problem specific parameters, its values, events sequence and conditions can be used as conditional inputs to the model and the model can generate the unseen problem set 240.

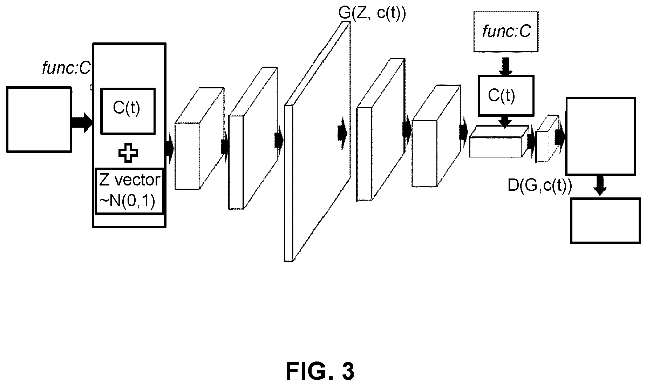

A conditional Deep Convolution Generative Adversarial Network (DCGAN) model 230 is trained to achieve this where known problem specific parameters, its values, events sequence will be the conditional parameters to guide new, unseen problem generation and integrate the domain knowledge. By way of example, Fig. 3 depicts how the parameters, its values, events sequence tuple is combined with the noise vector as conditional parameters for the model and the domain specific knowledge is used in creating new problem set.

(iii) Validation of generated problem set:
It is important to validate the generated problem set to decide 250 whether it is a new unseen problem, or a new path leads to a known problem. The generated (GAN model) combination of parameters, its values and also the generated sequence of events can be evaluated 250 with respect to known problems events sequence and the parameters combination (which was derived in step (i)). Based on the evaluation results the decision can be taken whether the generated fault is a new unseen fault or a same fault (i.e. known first fault) with different path (i.e. sequence of events). Similarity measure techniques (such as cosine similarity) can be used for this evaluation purpose.

(iv) Prepare solution for unseen problem 260:
Once the new unseen problem set it identified, the solution to those issues can be devised beforehand, so that the service engineers should have the knowledge of those issues and have solution ready for such issues. These derived unseen problems or new paths to the known problems can be taken in consideration (where possible) during the development/deployment phase of the medical equipment. (v) Feed New Problem and Solution to Next Development Cycle:

The new fault and corresponding solution that is devised on existing system is fed 270 to next development cycle where a next generation development is taking place. This enables removal of the said fault by pro-actively addressing it in the design/development phase. This makes system more robust as new generation of device is produced.

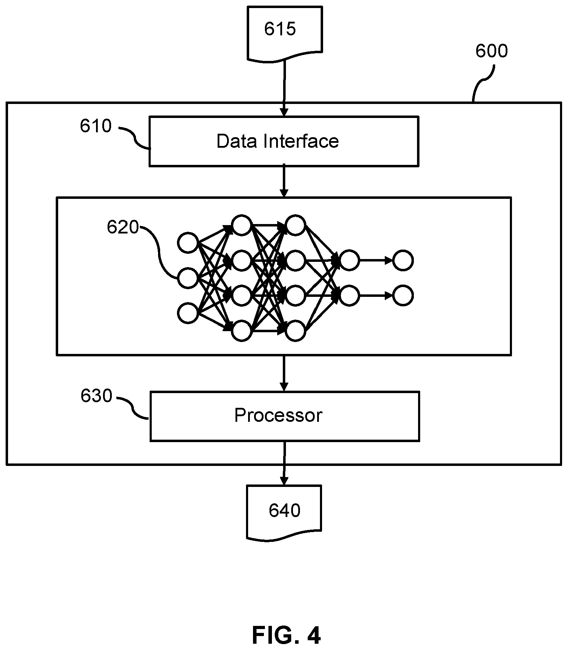

By way of summary of another proposed implementation, Fig. 4 depicts a simplified block diagram of a system 600 for predicting faults of a medical device.

The system comprises a data interface 610 that is configured to obtain a first set 615 of contributing factors that led to a first fault of the medical device. Specifically, the data interface 610 comprises an analysis component configured to obtain problem data describing the first fault of the medical device and to analyzes the problem data to determine a first set of contributing factors that led to the fault of the medical device via a first path of events.

A machine learning algorithm 620 of the system 600 is configured to receive a description of the first fault of the medical device and the first set of contributing factors problem data and the log file browsing data. As detailed above, the description of the first fault may comprise a textual description of the fault provided by a user. The machine learning algorithm 630 is trained to output a second set of contributing factors that will lead to an occurrence of the first fault of the medical device.

The system 600 also comprises a processor 630 that is configured to analyzes the second set of contributing factors and the first fault of the medical device to determine if the second set of contributing factors leads to an occurrence of the first fault. The processors 630 is also configured to determine, responsive to determining that the second set of contributing factors does not lead to an occurrence of the first fault, a predicted fault of the medical device that is led to by the second set of contributing factors.

The description 640 of the second set of contributing factors and the predicted fault is provided as an output 640 from the system 600, and may thus be used for predicting and analyzing unforeseen faults of the medical device.

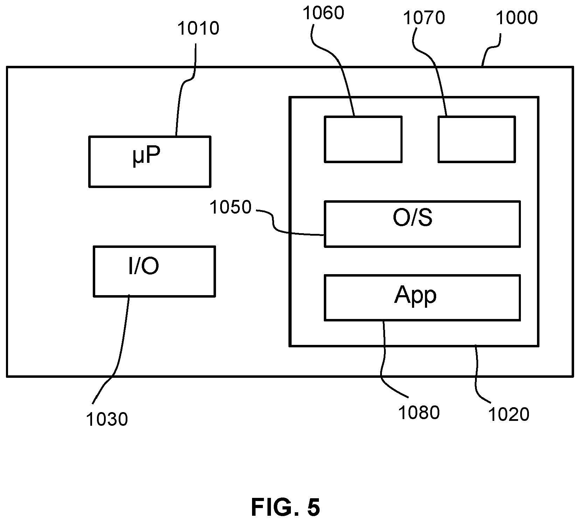

Fig. 5 illustrates an example of a computer 1000 within which one or more parts of an embodiment may be employed. Various operations discussed above may utilize the capabilities of the computer 1000. For example, one or more parts of a system for predicting faults of a medical device may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 10 10, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-3 and the systems of Figs. 4-5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figs. 4-5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method for predicting faults of a medical device, the method comprising:
obtaining a first set of contributing factors that led to a first fault of the medical device;
inputting a description of the first fault of the medical device and the first set of contributing factors to a machine learning algorithm, the machine learning algorithm being trained to predict a predicted second set of contributing factors that will lead to an occurrence of the first fault of the medical device, the second set of contributing factors being different to the first set of contributing factors; and
outputting the second set of contributing factors.

2. The method of claim 1, further comprising:
analyzing the second set of contributing factors and the first fault of the medical device to determine if the second set of contributing factors leads to an occurrence of the first fault; and
responsive to determining that the second set of contributing factors does not lead to an occurrence of the first fault, determining a predicted fault of the medical device that is led to by the second set of contributing factors, wherein the predicted fault is different from the first fault.

3. The method of claim 2, wherein analyzing comprises:
determining a value of a similarity measure between the first set of contributing factors attributes and the second set of contributing factors; and
based on the determining value of a similarity measure, determining if the second set of contributing factors leads to an occurrence of the first fault.

4. The method of claim 2, wherein analyzing comprises:
representing the first and second set of contributing factors and the first fault as connected graphs;
comparing the connected graphs; and
based on the comparison result, determining if the second set of contributing factors leads to an occurrence of the first fault.

5. The method of any of claims 2 to 4, further comprising:
determining a solution to the predicted fault of the medical device.

6. The method of any of claims 1 to 5, wherein the machine learning algorithm is trained using a training algorithm configured to receive an array of training inputs and respective known outputs, wherein a training input comprises: problem data describing a known fault of the medical device and an associated set of contributing factors that lead to an occurrence of the known fault of the medical device; and wherein a respective known output comprises a different set contributing factors that lead to an occurrence of the known fault of the medical device.

7. The method of any of claims 1 to 6, wherein obtaining the first set of contributing factors comprises:
obtaining problem data describing the fault of the medical device; and
analyzing the problem data to determine a first set of contributing factors that led to the fault of the medical device.

8. The method of any of claims 1 to 7, wherein obtaining the first set of contributing factors comprises:
analyzing an event history of the medical device.

9. The method of any of claims 1 to 8, wherein obtaining the first set of contributing factors comprises:
receiving, via in input interface, a description of contributing factors provided by a respondent in response to a request for data,
and optionally wherein the request for data comprises a fault analysis questionnaire.

10. The method of any of claims 1 to 9, wherein obtaining the first set of contributing factors comprises:
performing a natural language processing analysis on the description of the fault of the medical device.

11. The method of any of claims 1 to 10, wherein the machine learning algorithm comprises a generative adversarial network.

12. A computer program comprising code means for implementing the method of any of claims 1 to 11 when said program is run on a processing system.

13. A system for predicting faults of a medical device, the system comprising:
a data interface configured to obtain a first set of contributing factors that led to a first fault of the medical device; and
a machine learning algorithm configured to receive a description of the first fault of the medical device and the first set of contributing factors, the machine learning algorithm being trained to output predicted second set of contributing factors that will lead to an occurrence of the first fault of the medical device.

14. The system of claim 13, further comprising:
a processor arrangement configured to analyze the second set of contributing factors and the first fault of the medical device to determine if the second set of contributing factors leads to an occurrence of the first fault; and to determine responsive to determining that the second set of contributing factors does not lead to an occurrence of the first fault, a predicted fault of the medical device that is led to by the second set of contributing factors, wherein the predicted fault is different from the first fault.

15. The system of claim 10, wherein the data interface comprises an analysis component configured to obtain problem data describing the fault of the medical device and to analyze the problem data to determine a first set of contributing factors that led to the fault of the medical device via a first path of events.
